# EUROPEAN PATENT APPLICATION

(11) **EP 2 848 941 A1**
(43) Date of publication of application: **18.03.2015**
(21) Application number: 13788211.4
(22) Date of filing: 07.05.2013
(51) Int. Cl.: G01N 33/579

(54) **METHOD FOR MEASURING ORGANISM-ORIGINATED PHYSIOLOGICALLY ACTIVE SUBSTANCE IN HYALURONIC ACID PREPARATION**

(30) Priority: 09.05.2012 JP 2012107314
(71) Applicant: Kowa Company Ltd., Naka-ku Nagoya-shi Aichi 460-8625 (JP)
(72) Inventor: HIRONO, Taisuke, Higashimurayama-shi Tokyo 189-0022 (JP); SUGIURA, Yuka, Higashimurayama-shi Tokyo 189-0022 (JP)
(74) Representative: Henderson, Helen Lee
(86) International application number: PCT/JP2013/062825
(87) International publication number: WO 2013/168695

(57) **Abstract**

Provided is a technique for achieving a higher measurement accuracy in the detection of an organism-originated physiologically active substance or the measurement of the concentration of the organism-originated physiologically active substance in a hyaluronic acid preparation. The present invention relates to a measurement method for detecting an organism-originated physiologically active substance or measuring the concentration of the organism-originated physiologically active substance in a hyaluronic acid preparation by mixing the hyaluronic acid preparation with an AL reagent to allow the organism-originated physiologically active substance in the hyaluronic acid preparation and an AL to react with each other. A mixed solution of the hyaluronic acid preparation with the AL reagent is diluted with an aqueous electrolyte solution, and a specific protein having a higher affinity for the organism-originated physiologically active substance than that of hyaluronic acid is added to the mixed solution.

## Description

### TECHNICAL FIELD

The present invention relates to a measuring method in order to detect a physiologically active substance such as endotoxin or β-D-glucan in a sample containing the physiologically active substance which is derived from an organism and has a characteristic to gel by the reaction with an AL or to measure the concentration thereof, and particularly it relates to a method to detect an organism-originated physiologically active substance in a hyaluronic acid preparation or to measure the concentration thereof.

### BACKGROUND ART

Endotoxin is lipopolysaccharides present in the cell wall of Gram-negative bacteria and the most representative pyrogenic substance. There is a concern that a severe side effect such as fever or shock is caused when an infusion solution, an injection drug, a dialysis solution, blood, or the like that is contaminated with this endotoxin is injected into the human body. For this reason, the above pharmaceutical preparations and the like are required to be managed so as not to be contaminated by endotoxin.

Meanwhile, a serine protease that is activated by endotoxin is present in the hemocyte extract of horseshoe crab (hereinafter, also referred to as the "AL: Amoebocyte Lysate"). Moreover, coagulogen present in the AL is hydrolyzed into a coagulin by an enzymatic cascade by the activated serine protease in accordance with the amount of endotoxin when the AL reacts with endotoxin, and associated to produce an insoluble gel. Using such a characteristic of AL enables to detect endotoxin with high sensitivity.

In addition, β-D-glucan is polysaccharides (polysaccharides) constituting a cell membrane which is characteristic for fungi. It is possible to detect not only fungi, such as Candida, Aspergillus, or Cryptococcus, which is commonly seen in a general medical practice but also the existence of rare fungi by measuring β-D-glucan and thus an extensive screening of fungal infection is possible.

In this case also, it is possible to detect β-D-glucan with high sensitivity by using the characteristic that the hemocyte extract component AL of horseshoe crab is coagulated (gel coagulation) by β-D-glucan.

The following methods can be exemplified as a specific method for the case of performing the detection of an organism-originated physiologically active substance such as an endotoxin or β-D-glucan (hereinafter, also referred to as the "predetermined physiologically active substance") by the hemocyte extract component AL of horseshoe crab or the measurement of the concentration thereof. First, there is a semiquantitative gelation reversing method in which a mixture prepared by mixing the AL and a sample to be subjected to the detection of the predetermined physiologically active substance or the concentration measurement thereof (hereinafter, also simply referred to as the "measurement of the predetermined physiologically active substance") is left to stand, the container is reversed in a certain period of time, and whether the gelation has proceeded or not is determined by the presence or absence of falling of the mixture, thereby examining if the predetermined physiologically active substance is contained in the sample at a certain concentration ormore. In addition, there is the turbidimetric method to analyze by measuring the time course of the turbidity of the sample brought with the gel formation by the reaction of the AL with the predetermined physiologically active substance, the colorimetery to measure the change in color of the sample using a synthetic substrate that is hydrolyzed by the enzymatic cascade and thus exhibits a color, or the like.

In the case of performing the measurement of the predetermined physiologically active substance by the turbidimetric method described above, a mixture of the sample for measurement and the AL is produced in a glass measuring cell which is dry-heat sterilized. Thereafter, light is irradiated from the outside for the gelation of the mixture, and the change in transmittance of the mixture caused by the gelation is opticallymeasured. On the contrary to this, as a method capable of measuring the predetermined physiologically active substance in a shorter period of time, a light scattering method (laser beam scattering particle counting method) has been proposed in which the mixture of the sample for measurement and the AL is stirred using, for example, a magnetic stirring bar to produce gel particles and then the peak number of the laser beam scattered by the gel particles is detected. In the same manner, the stirring turbidimetric method has been proposed in which the turbidity of the sample due to the gel particles produced by stirring the mixture of the sample for measurement and the AL is detected by the intensity of light passing through the mixture.

One of the problems when measuring endotoxin in a sample is that the measurement is affected by the interference (inhibition and promotion) of the sample. In other words, there is a case in which the measured value of endotoxin is lower than the actual measured value (inhibition) or a case in which the measured value of endotoxin is higher than the actual measured value (promotion) in contrast by the influence of the sample itself to be measured. It is considered that this often occurs because the reaction activity of AL, the physical properties of endotoxin, or the measurement system other than the main reaction is influenced by the characteristics or the like of the sample and thus the Limulus reaction that occurs when the sample is mixed with the AL does not efficiently proceed or the reaction excessivelyproceeds. Hence, a technique to stabilize endotoxin in a sample is required in order to measure endotoxin in the sample with high precision. With regard to this, a technique to allow albumin to coexist in order to stabilize endotoxin in an aqueous solution is known, for example, in the concentration measurement of endotoxin in an aqueous solution such as a dialysis solution for hemodialysis (for example, see Patent Document 1).

However, it is known that a hyaluronic acid preparation is injected into the anterior chamber part in order to maintain the tension of the anterior chamber part of eyeball during the surgery in the cataract surgery, the intraocular lens insertion surgery, and the like. There is a case in which a situation called toxic anterior segment syndrome (TASS) that causes the inflammation of the anterior chamber part is brought about after the surgery when this hyaluronic acid preparation is contaminated with endotoxin.

To cope with this, a regulation in which the permissible contamination value of endotoxin in the hyaluronic acid preparation is 0.1 EU/mL has been proposed in ISO and FDA, and thus the concentration of endotoxin contained in the hyaluronic acid preparation is required to be precisely measured. However, it is difficult to measure endotoxin in the hyaluronic acid preparation with high precision in some cases since the viscosity is high and/or the Limulus reaction is inhibited in particular.

With regard to this, a technique is known in which the concentration of endotoxin is measured by diluting the hyaluronic acid preparation at 30°C or higher and 40°C or less with water for dilution in the same temperature range (for example, see Patent Document 2). However, the molecular weight of the hyaluronic acid preparation varies from high molecular weight to low molecular weight, and thus a sufficiently stable measurement is possible by the above technique in the case of a hyaluronic acid preparation such as Viscoat (registered trademark) (500 in molecular weight) but a stable measurement of the concentration of endotoxin contained in a hyaluronic acid preparation having a high viscosity such as Healon (registered trademark) (1900 to 3900 in molecular weight) or Healon V (4000 in molecular weight) is still difficult.

### CITATION LIST

### PATENT LITERATURE

Patent Literature 1: JP 2817606 B1
Patent Literature 2: WO 2012/029171 A

### SUMMARY OF INVENTION

### TECHNICAL PROBLEM

The invention has been devised in view of the above problem, and an object thereof is to provide a technique capable of obtaining higher measurement precision by suppressing the inhibitory action of hyaluronic acid in the detection of an organism-originated physiologically active substance in a hyaluronic acid preparation or the measurement of the concentration thereof.

### SOLUTION TO PROBLEM

The invention relates to a measuring method to detect an organism-originated physiologically active substance in a hyaluronic acid preparation or to measure the concentration thereof by mixing a hyaluronic acid preparation with an AL reagent and allowing an organism-originated physiologically active substance in the hyaluronic acid preparation to react with an AL. In addition, the invention is characterized in the greatest in that the inhibitory effect of hyaluronic acid is suppressed by containing a predetermined protein exhibiting higher affinity for the organism-originated physiologically active substance than hyaluronic acid as well as diluting the mixture of a hyaluronic acid preparation and an AL reagent with an aqueous electrolyte solution.

More specifically, the invention is a method of measuring an organism-originated physiologically active substance to measure a concentration of a physiologically active substance in a hyaluronic acid preparation by allowing the organism-originated physiologically active substance present in the hyaluronic acid preparation to react with an AL of the hemocyte extract of horseshoe crab, and in the method, an aqueous electrolyte solution for dilution and a predetermined protein exhibiting higher affinity for the organism-originated physiologically active substance than hyaluronic acid are contained in a mixture of an AL reagent containing the AL and the hyaluronic acid preparation.

Here, in the reaction of the organism-originated physiologically active substance in the hyaluronic acid preparation with the AL in the AL reagent, it has been found that hyaluronic acid itself has a function of inhibiting the reaction of the organism-originated physiologically active substance with the AL. Moreover, it has been found that hyaluronic acid itself inhibits the aggregation of coagulin by the reaction of the organism-originated physiologically active substance with the AL. It has been considered that this is because the organism-originated physiologically active substance is incorporated into the molecule of hyaluronic acid and thus the organism-originated physiologically active substance and the AL are in a state difficult to react with each other, and a charged hyaluronic acid molecule enters between the coagulins to couple with one another and thus inhibits the electrical coupling of coagulins.

To cope with this, in the invention, first, the hyaluronic acid preparation is diluted using an aqueous electrolyte solution. By virtue of this, it is possible to suppress the inhibition of the electrical coupling of coagulins by the charged hyaluronic acid molecule. Moreover, in the invention, a predetermined protein exhibiting higher affinity for the organism-originated physiologically active substance than hyaluronic acid is contained in the mixture of the hyaluronic acid preparation and the AL reagent. By virtue of this, it is possible to take out the organism-originated physiologically active substance that is incorporated in the molecule of hyaluronic acid and thus to form a state in which the organism-originated physiologically active substance easily reacts with the AL.

As a result, it is possible to promote the reaction of the organism-originated physiologically active substance in the hyaluronic acid preparation with the AL and to promote the gelation of coagulin due to the reaction, and thus it is possible to increase the measurement sensitivity of the organism-originated physiologically active substance in the hyaluronic acid preparation and to improve the measurement precision.

In addition, in the invention, the predetermined protein may be albumin. Here, the behavior of albumin with respect to the surrounding pH has been found in detail. Hence, it is possible to more easily control the reaction of the organism-originated physiologically active substance in the hyaluronic acid preparation with the AL by using albumin as the predetermined protein and further controlling the pH of the mixture of the hyaluronic acid preparation and the AL reagent.

In addition, in the invention, a phosphate buffered saline may be used as the aqueous electrolyte solution for dilution. Negative charges are more abundantly contained in this phosphate buffered saline, and thus it is possible to more reliably suppress the inhibition of the electrical coupling of coagulins by the charged hyaluronic acid molecule by using the phosphate buffered saline as the aqueous electrolyte solution for phosphoric acid dilution. Moreover, it is possible to more stabilize the pH of the mixture of the hyaluronic acid preparation and the AL reagent and thus to more easily control the characteristics of the predetermined protein in the mixture of the hyaluronic acid preparation and the AL reagent.

In addition, in the invention, a pH of the mixture of the AL reagent and the hyaluronic acid preparation in the state of containing the aqueous electrolyte solution for dilution may be set to be 5.5 or higher. Here, it has been found that albumin forms a chain-shaped fragment in a case in which the surrounding pH thereof is higher than the isoelectric point of albumin, and thus it is possible to form a state in which the organism-originated physiologically active substance that is adsorbed to albumin easily reacts with the AL. On the other hand, it has been found that albumin aggregates in a case in which the surrounding pH is lower than the isoelectric point of albumin, the organism-originated physiologically active substance is incorporated into albumin, and thus the organism-originated physiologically active substance and the AL are in a state difficult to react with each other. In addition, it has been found that the isoelectric point of albumin is about from 4.4 to 5.3.

Hence, it is possible to more reliably maintain the pH of the mixture in a state of being higher than the isoelectric point of albumin by setting the pH of the mixture of the AL reagent and the hyaluronic acid preparation in the state of containing the aqueous electrolyte solution for dilution to 5.5 or higher. As a result, it is possible to maintain a state in which the organism-originated physiologically active substance easily reacts with the AL and thus to perform the detection of the organism-originated physiologically active substance and the measurement of the concentration thereof with higher precision.

In addition, in the invention, the organism-originated physiologically active substance may be endotoxin or β-D-glucan.

In that case, it is possible to more accurately perform the detection of endotoxin that is the most representative pyrogenic substance or the measurement of the concentration thereof, and it is possible to suppress that an infusion solution, an injection drug, a dialysis solution, blood, or the like that is contaminated with endotoxin is injected into the human body and thus a side effect is caused. In the same manner, it is possible to more accurately perform the detection of β-D-glucan or the measurement of the concentration thereof and an extensive screening of fungal infection including not only fungi, such as Candida, Aspergillus, or Cryptococcus, which is commonly seen in a general medical practice but also rare fungi.

Meanwhile, the means for solving the problem of the invention described above can be used in combination as possible. Moreover, it is possible to exemplify globulin, lysozyme, and the like in addition to albumin as the predetermined protein in the invention.

### EFFECT OF INVENTION

According to the invention, it is possible to obtain higher measurement precision in the detection of an organism-originated physiologically active substance in a hyaluronic acid preparation or the measurement of the concentration thereof.

### BRIEF DESCRIPTION OF DRAWINGS

Fig. 1 is a diagram for describing the difference in the behavior of an albumin molecule depending on the pH of the mixture of a hyaluronic acid preparation and an AL reagent.
Fig. 2 is a graph illustrating the relationship between the pH of the mixture and the measurement result by the laser beam scattering particle counting method in the case of adding albumin to the mixture of a hyaluronic acid preparation and an AL reagent.
Fig. 3 is a graph illustrating the relationship between the pH of the mixture and the recovery rate of endotoxin in the case of adding albumin to the mixture of a hyaluronic acid preparation and an AL reagent.
Fig. 4 is a graph illustrating the relationship between the change in pH of the mixture and the change in the recovery rate of endotoxin in the case of adding albumin to the mixture of a hyaluronic acid preparation and an AL reagent.
Fig. 5 is a diagram illustrating a schematic configuration of a laser beam scattering particle measuring apparatus according to Example of the invention.
Fig. 6 is a schematic diagram for describing the gelation process of AL by endotoxin or β-D-glucan and the detection method thereof.

### DESCRIPTION OF EMBODIMENTS

The best mode for carrying out the invention will be described in detail with reference to the accompanying drawings. Meanwhile, in the following Example, endotoxin will be mainly described as an example as an organism-originated physiologically active substance, but it is a matter of course that the invention can also be applied to other physiologically active substances derived from organisms, for example, β-D-glucan and the like.

### [Example 1]

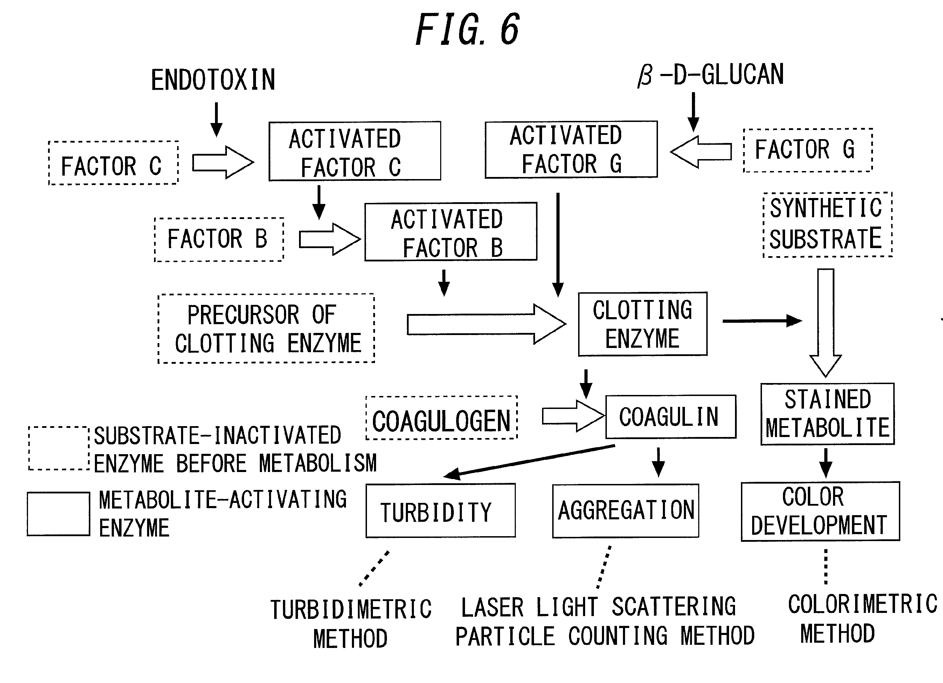

The process has been well investigated in which the AL reacts with endotoxin to produce a gel. In other words, it is believed that, as illustrated in Fig. 6, the factor C is activated to be an activated factor C when endotoxin couples with the factor C that is a serine protease in the AL, and the factor B that is another serine protease in the AL is hydrolyzed and activated by the activated factor C to be an activated factor B. The precursor of clotting enzyme in the AL is immediately hydrolyzed by this activated factor B to be the clotting enzyme, and further the coagulogen in the AL is hydrolyzed by this clotting enzyme to produce coagulin. Thereafter, the coagulins thus produced are associated with one another to further produce an insoluble gel, and the entire AL is involved in this to be a gel.

In addition, in the same manner, the factor G is activated to be an activated factor G when β-D-glucan couples with the factor G in the AL, and the precursor of clotting enzyme in the AL is hydrolyzed by this activated factor G to be the clotting enzyme. As a result, in the same manner as the reaction of endotoxin with the AL, coagulin is produced, and the coagulins thus produced are associated with one another to further produce an insoluble gel.

This series of reactions is similar to the process of producing a fibrin gel via a serine protease such as the Christmas factor or thrombin found in a mammal. Such an enzymatic cascade reaction exhibits a significantly strong amplification action even with a significantly small amount of an activating factor since the subsequent cascades are sequentially activated. Hence, according to the method of measuring a predetermined physiologically active substance using an AL, it has been possible to detect a predetermined physiologically active substance in a significantly small amount of a subpicogram/mL order.

The turbidimetric method and the laser beam scattering particle counting method are exemplified as a measuring method capable of quantifying the predetermined physiologically active substance as described above. As illustrated in Fig. 6, these measuring methods allows a high sensitive measurement as the former detects the aggregate of the coagulin produced by the enzymatic cascade reaction of this AL as the turbidity of the sample and the latter detects the aggregate of the coagulin as the fine particles of the gel produced in the system.

Particularly, the laser beam scattering particle counting method exhibits higher sensitivity than the turbidimetric method since the fine particles of the gel produced in the system are directly measured, and it can detect the production of gel in a shorter time compared to the turbidimetric method since generally the sample consisting of an AL and a specimen is forcedly stirred.

Next, a specific example with regard to the effect on the human body by endotoxin will be described. It is known that a hyaluronic acid preparation is injected into the anterior chamber part in order to maintain the tension of the anterior chamber part of eyeball during the surgery, such as the cataract surgery and the intraocular lens insertion surgery, in which the removal of the aqueous humor of eyeball is presupposed. There is a case in which a situation to develop toxic anterior segment syndrome (TASS) and thus to cause the inflammation of the anterior chamber part is brought about after the surgery when this hyaluronic acid preparation is contaminated with endotoxin.

To cope with this, a regulation in which the permissible contamination value of endotoxin in the hyaluronic acid preparation is 0.1 EU/mL has been proposed by ISO and FDA, and thus the concentration of endotoxin contained in the hyaluronic acid preparation is required to be precisely measured. However, it is difficult to measure endotoxin in the hyaluronic acid preparation with high precision in some cases since the viscosity of hyaluronic acid is significantly high and thus it is difficult to uniformly mix the AL reagent with the hyaluronic acid preparation when the concentration of endotoxin is measured using the AL.

The molecular weights of hyaluronic acid preparations varies as presented in Table 1, particularly the hyaluronic acid having a high molecular weight becomes a state of having a higher viscosity, and thus it is more difficult to measure endotoxin in the hyaluronic acid preparation with high precision in some cases.

**[Table 1]**

| Hyaluronic acid | Healon V (Registered trademark) | Healon (Registered trademark) | Opelead (Registered trademark) | Opegan (Registered trademark) | Viscoat (Registered trademark) |
|---|---|---|---|---|---|
| Molecular weight(KDa) | 4000 | 1900-3900 | 1530-2130 | 600-1200 | 500 |

In addition, the relationship between the dilution rate of hyaluronic acid and the recovery rate in the measurement of endotoxin is presented in Table 2. Here, the recovery rate represents the ratio of the amount of endotoxin detected to the amount of endotoxin added to the endotoxin-free hyaluronic acid preparation, and it is regarded that the measurement is performed with significantly high precision when the recovery rate is 100%.

**[Table 2]**

| Dilution rate of hyaluronic acid | 20 times (0.005EU/mL) | 30 times (0.003EU/mL) | 40 times (0.0025EU/mL) | 100 times (0.001EU/mL) |
|---|---|---|---|---|
| Recovery rate(%) | 54.2±7.6 | 63.3±0.5 | 46.5±7.7 | 95.0±11.3 |

As can be seen from Table 2, it is impossible to obtain a satisfactory recovery rate unless diluted about 100 times when hyaluronic acid is diluted using dilution water. However, it has been a present situation that a significant improvement in measurement precision of endotoxin cannot be expected when the hyaluronic acid preparation is simply diluted as great as 100 times with dilution water since the content of endotoxin itself in the measurement system is lowered.

Furthermore, by intensive investigations of the inventors, it has been found that in addition to the effect of viscosity as described above, the action of hyaluronic acid itself to inhibit the reaction of the endotoxin with the AL and the action of hyaluronic acid itself to inhibit the gelation of coagulin are involved in the cause that makes the measurement of endotoxin in the hyaluronic acid preparation difficult. This action is considered as follows.

Here, in the measurement of endotoxin in the hyaluronic acid preparation, first endotoxin and the AL present in a reactive manner in the mixture come close to each other to result in the reaction. Thereafter, in the process in which coagulogen is hydrolyzed by the reaction of endotoxin and the AL to produce coagulin and these coagulins are associated with one another to further produce an insoluble gel, first, coagulins couple with one another in a chain shape. Thereafter, the coagulins coupled in this chain shape are further coupled by a hydrophobic bonding or an electrical coupling to form a gel.

In this process, the action of hyaluronic acid itself to inhibit the reaction of the endotoxin with an AL is considered that endotoxin in the mixture of the hyaluronic acid preparation and the AL reagent is in a state of being incorporated in the molecule of hyaluronic acid, the opportunity for endotoxin and the AL to come close to each other is deprived, and thus the reaction between endotoxin and the AL is inhibited. In addition, the action of hyaluronic acid itself to inhibit the gelation of coagulin is considered that a charged hyaluronic acid is present between the coagulins that are going to couple with one another and thus the electrical coupling of the coagulins is inhibited.

To cope with this, in the present Example, first, the molecule of hyaluronic acid is electrically stabilized (or the balance between the hydrophobic bonding and the electrical coupling in the coupling of coagulins is changed) by using an aqueous electrolyte solution as the dilution liquid when diluting the hyaluronic acid preparation, and thus it is suppressed that the charged hyaluronic acid molecule inhibits the electrical coupling of coagulins coupled in a chain shape. By virtue of this, it is possible to promote the association and gelation of the coagulins when endotoxin reacts with the AL in the mixture of the hyaluronic acid preparation and the AL reagent.

In Table 3, the difference in recovery rate of endotoxin in the case of using water (Otsuka distilled water manufactured by Otsuka Pharmaceutical Factory, Inc., the same applies in the present Example) as a dilution liquid of the hyaluronic acid preparation and the case of using a NaCl solution (0.1 M, manufactured by Wako Pure Chemical Industries, Ltd., the same applies in the present Example) and phosphate buffered saline (hereinafter, also referred to as PBS) (0.01 M, pH 7.4, manufactured by Nacalai Tesque, Inc., the same applies in the present Example) is presented.

**[Table 3]**

| Dilution rate of hyaluronic acid | | 20 times (0.005EU/mL) | 30 times (0.003EU/mL) | 40 times (0.0025EU/mL) | 100 times (0.001EU/mL) |
|---|---|---|---|---|---|
| Recovery rate(%) | WFI (water) | 54.2±7.6 | 63.3±0.5 | 46.5±7.7 | 95.0±11.3 |
| | NaCl | - | - | 66.1±3.6 | - |
| | PBS | - | - | 77.1±9.5 | 102.5±11.7 |

As can be seen from Table 3, it can be suppressed that the charged hyaluronic acid molecule inhibits the gelation of coagulin by diluting the hyaluronic acid preparation using an aqueous electrolyte solution such as a NaCl solution or PBS, and thus it is possible to improve the recovery rate of endotoxin (at a dilution rate of 40 times and 100 times in the Table). Meanwhile, PBS contains NaCl, KCl, Na₂HPO₄, KH₂PO₄, and the like and has a large number of negative charges, and thus it can be said that PBS is more suitable for the purpose of the present Example.

In addition, in the present Example, human serum albumin (HSA: hereinafter, also simply referred to as albumin) is added to the mixture of the hyaluronic acid preparation and the AL reagent (Limulus ES-II Single Test Wako (manufactured by Wako Pure Chemical Industries, Ltd.): the same applies in the present Example) when measuring endotoxin contained in the hyaluronic acid preparation. The reason for this is as follows. Endotoxin in the hyaluronic acid preparation is in a state of being incorporated in the molecule of hyaluronic acid as described above and the reaction of endotoxin with the AL is inhibited since the approach opportunity thereof is deprived. However, it is believed that albumin pulls endotoxin incorporated in hyaluronic acid apart when albumin is added in this state since albumin exhibits higher affinity for endotoxin compared to hyaluronic acid, and thus endotoxin can be in a state easy to react with AL.

In Table 4, the recovery rate of endotoxin in a case in which the hyaluronic acid preparation is diluted using water or an aqueous electrolyte solution and further albumin is added is presented.

**[Table 4]**

| | Dilution rate of hyaluronic acid | | 20 times (0.005EU/mL) | 30 times (0.003EU/mL) | 40 times (0.0025EU/mL) | 100 times (0.001EU/mL) |
|---|---|---|---|---|---|---|
| Recovery rate(%) | WFI (water) | Without albumin | 54.2±7.6 | 63.3±0.5 | 46.5±7.7 | 95.0±11.3 |
| | | Albumin added | - | - | 33.5±5.8 | 92.7±14.7 |
| | NaCl | Without albumin | - | - | 66.1±3.6 | - |
| | | Albumin added | - | 104.1±4.8 | 91.8±7.2 | - |
| | PBS | Without albumin | - | - | 77.1±9.5 | 102.5±11.7 |
| | | Albumin added | 79.4±8.8 | 106.3±10.7 | 113.0±6.4 | 81.4±2.8 |

As can be seen from Table 4, it is possible to obtain a high recovery rate even in the case of having a dilution rate of from 20 times to 40 times by diluting the hyaluronic acid preparation with an aqueous electrolyte solution such as a NaCl solution or the phosphate buffered saline (PBS) and further adding albumin.

Here, it has been demonstrated by the intensive investigations of the inventors that the effect of the case of adding albumin to the mixture of the hyaluronic acid preparation and the AL reagent as described above is greatly influenced by the pH of the mixture in the diluted state. In addition, it has been found that this is in a close relationship with the change in the behavior of albumin in accordance with the surrounding pH of albumin.

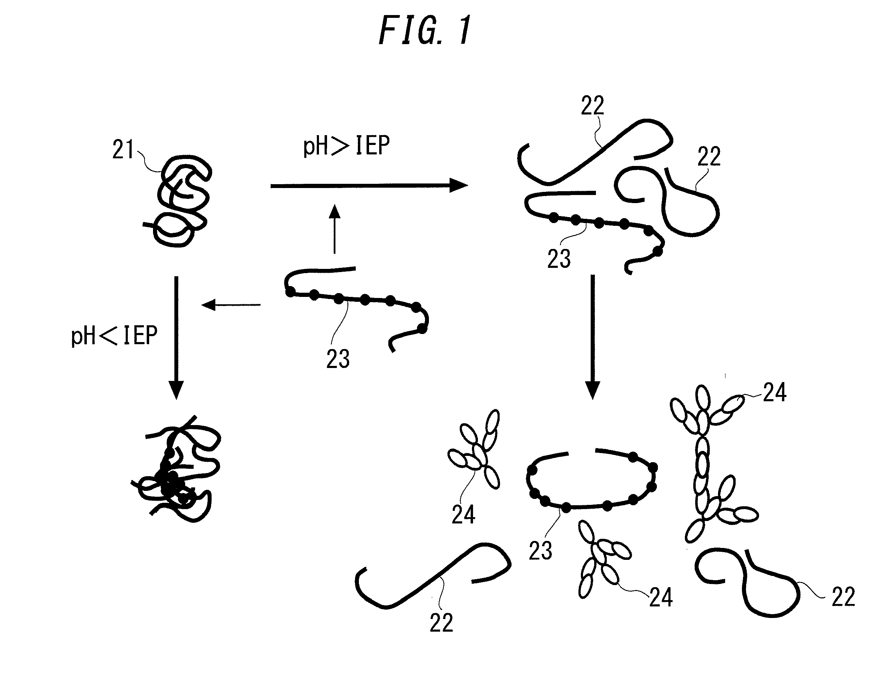

Fig. 1 is a diagram for explaining the change in the behavior of albumin in accordance with the value of the surrounding pH. In Fig. 1, 21 represents the molecule of albumin. The isoelectric point of albumin 21 (hereinafter, also referred to as IEP) is a value between approximately 4.4 and 5.3. In addition, it is found that albumins 21 themselves randomly aggregate into a ball shape in a case in which the surrounding pH is lower than the isoelectric point of albumin 21. Hence, endotoxin 23 is in a state of being incorporated into the aggregate of albumin 21 even if endotoxin 23 is present in this state, and endotoxin 23 is in a state hard to react with the AL as well.

On the other hand, it is found that albumins 21 do not randomly aggregate but form a chain-shaped fragment 22 in a case in which the value of the surrounding pH is higher than the isoelectric point, and thus albumins 21 exist to align in a line. Hence, endotoxin 23 is not incorporated into the chain-shaped fragment 22 of albumin when endotoxin 23 is present in this state but can maintain the state of being exposed to the outside. In that case, endotoxin 23 is not prevented from associating with the factor C of AL and thus it is possible to form a state in which endotoxin 23 easily reacts with the AL. As a result, it becomes a state in which a coagulin gel 24 is easily produced by the reaction of endotoxin 23 with the AL.

Consequently, the reaction of endotoxin in the hyaluronic acid preparation with the AL in the AL reagent is inhibited, the start time of the reaction is delayed, and thus the recovery rate of endotoxin is also lowered in a case in which the hyaluronic acid preparation is diluted using an aqueous electrolyte solution and mixed with an AL reagent, and further the pH in the state of being added with albumin is lower than the isoelectric point of albumin. On the other hand, the reaction of endotoxin in the hyaluronic acid preparation with the AL in the AL reagent is promoted, the start time of the reaction is shortened, and thus the recovery rate of endotoxin is also improved in a case in which the pH in the above state is higher than the isoelectric point of albumin.

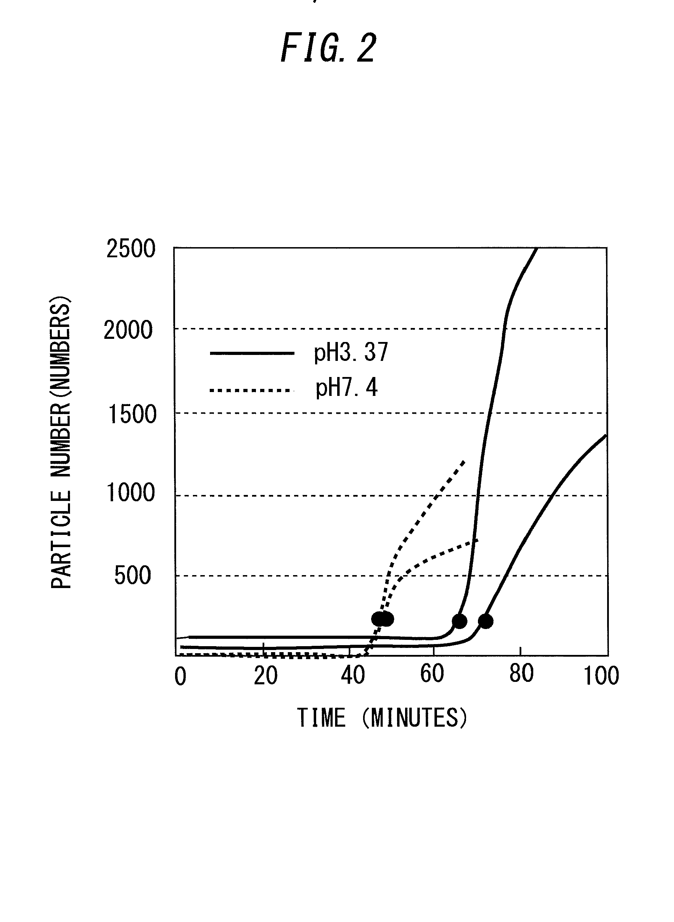

In Fig. 2, the result is illustrated which is obtained by preparing a mixture having a pH of the mixture of the hyaluronic acid preparation and the AL reagent in the state being diluted using an aqueous electrolyte solution of 3.37 and lower than the isoelectric point of albumin and a mixture having a pH of 7.4 and higher than the isoelectric point of albumin, and measuring the number of gel particles produced in both of the mixtures by the laser beam scattering particle counting method (using EX-300 manufactured by Kowa Company Ltd. , the same applies in the present Example). It can be seen that the increase in the number of gel particles in the mixture having a pH of 7.4 occurs earlier compared to the mixture having a pH of 3.37.

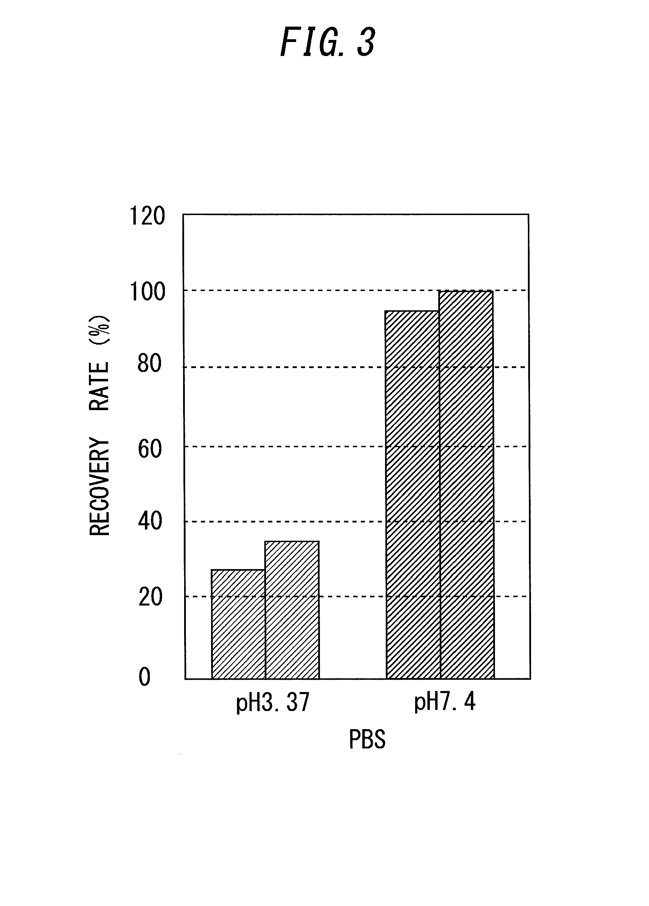

In addition, in Fig. 3, the recovery rate of endotoxin in a case in which the mixture has a pH of 3.37 and a case in which the mixture has a pH of 7.4 is illustrated. According to this, for both times in two times of measurement, the mixture having a pH of 7.4 achieves a recovery rate approximately 100% but the mixture having a pH of 3.37 only obtains a recovery rate approximately less than 40%. Consequently, it is comprehended that it is possible to improve the measurement precision of endotoxin by using a mixture having a pH higher than the isoelectric point of albumin.

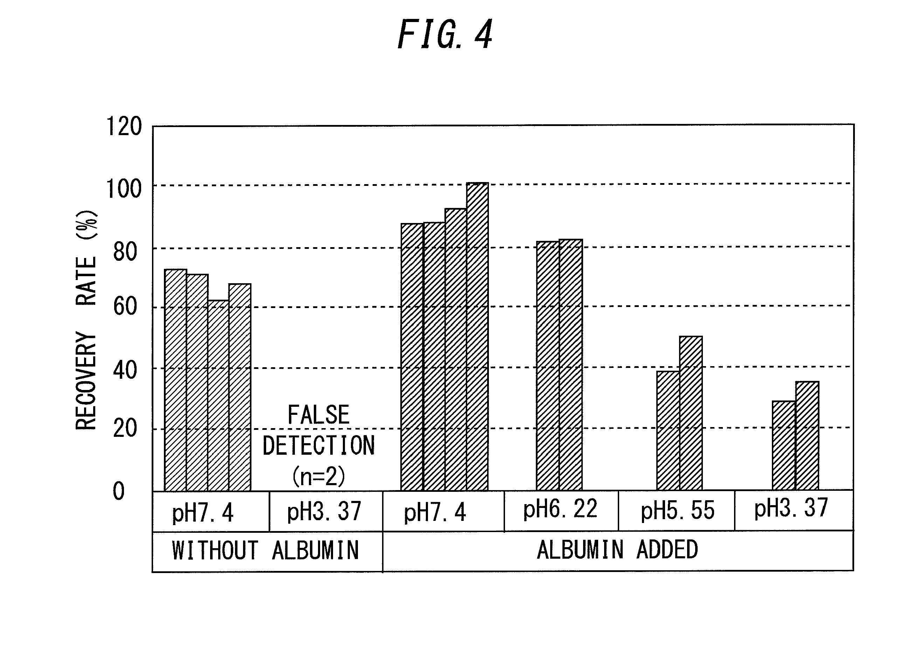

Furthermore, in Fig. 4, a graph is illustrated which verifies if the recovery rate of endotoxin is improved at any stage in the case of increasing the pH of the mixture of the hyaluronic acid preparation and the AL reagent. The recovery rate is improved to 80% or more when the pH exceeds from 5.5 to 6 for the items of albumin added in Fig. 4. The isoelectric point of albumin is about from 4.4 to 5.3 as described above, and thus the phenomenon described above has been proven more rigidly.

From the above results, in the present Example, it is possible to maintain the pH of the mixture higher than the isoelectric point of albumin by setting the pH of the mixture to 5.5 or higher in the case of adding albumin to the mixture of the hyaluronic acid preparation and the AL reagent, and thus it is possible to improve the measurement precision of endotoxin. In addition, it is possible to more reliably maintain the pH of the mixture higher than the isoelectric point of albumin by setting the pH of the mixture to 6.22 or higher, and thus it is possible to more reliably improve the measurement precision of endotoxin.

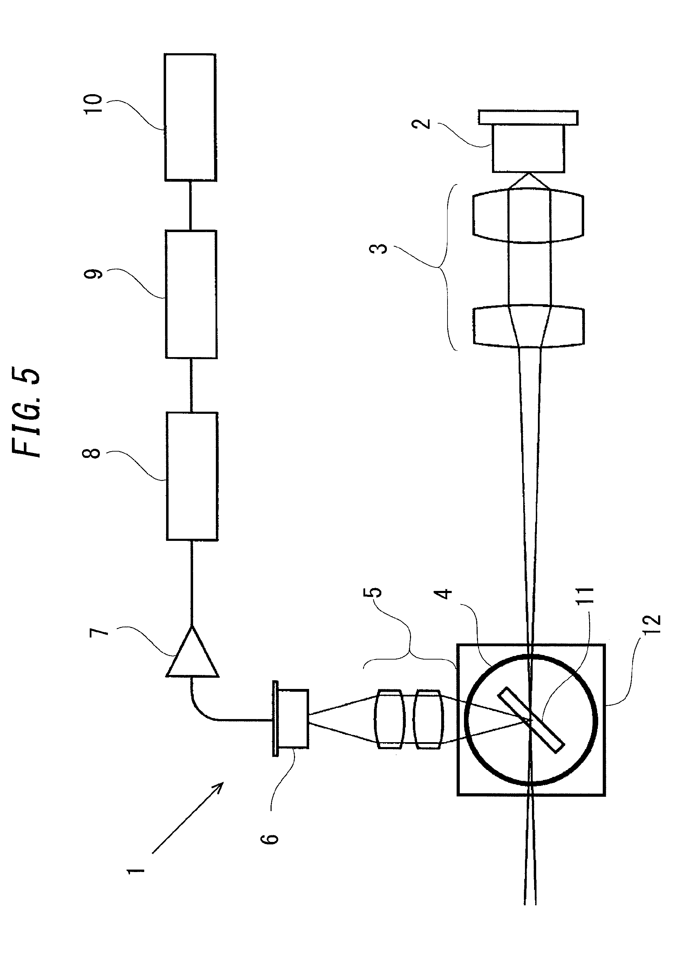

In Fig. 5, the schematic configuration of a light scattering particle measuring apparatus 1 as the apparatus for measuring endotoxin in the present Example is illustrated. A laser beam source is used as a light source 2 used in the light scattering particle measuring apparatus 1, but a super high luminance LED or the like may be used other than the laser beam source. The light irradiated from the light source 2 is focused by an incident optical system 3 and then incident on a sample cell 4. The mixture of the hyaluronic acid preparation and the AL reagent to be subjected to the measurement of endotoxin is held in this sample cell 4. The light incident on the sample cell 4 is scattered by the particles in the mixture (measuring objects such as a coagulin monomer and a coagulin oligomer).

An outgoing optical system 5 is disposed on the lateral side of the incident light axis of the sample cell 4. In addition, a light receiving element 6 which receives the scattered light which is scattered by the particles in the mixture in the sample cell 4 and focused by the outgoing optical system 5 and converts the scattered light into an electrical signal is disposed on the extension of the optical axis of the outgoing optical system 5. An amplifier circuit 7 to amplify the electrical signal photoelectrically converted by the light receiving element 6, a filter 8 for removing the noise from the electrical signal amplified by the amplifier circuit 7, an arithmetic and logic unit 9 which calculates the number of gel particles from the number of peaks of the electrical signal from which the noise is removed and further derives the concentration of endotoxin by determining the gelation detection time, and an indicator 10 to display the result are electrically connected to the light receiving element 6.

In addition, the sample cell 4 is equipped with a stirring bar 11 which is rotated by applying a magnetic force from the outside and stirs the mixture as a sample, and a stirrer 12 is equipped to the outside of the sample cell 4. By virtue of this, it is possible to adjust the presence or absence of stirring and the stirring speed.

In the light scattering particle measuring apparatus 1 described above, the appearance time of the coagulin gel particle (gelation detection time = gelation time) which is the final stage of the Limulus reaction is measured, and the concentration of endotoxin in a specimen is calculated using a calibration relationship established between the endotoxin concentration and the gelation detection time.

In the above Example, an example is described in which the invention is applied to the case of measuring the concentration of endotoxin by a laser beam scattering particle counting method using the light scattering particle measuring apparatus 1. It is a matter of course that the invention is applicable not only to the case by the laser beam scattering particle counting method but also to a case in which endotoxin in the hyaluronic acid preparation is measured by, for example, the turbidimetric method, the stirring turbidimetric method, and the colorimetric method.

Meanwhile, the substances, such as an aqueous electrolyte solution, hyaluronic acid, the AL reagent, and albumin, which are described in the above Example are not limited to the products and the products of the manufacturers used in the above, but it is a matter of course that the substances above can be replaced with anyone as long as it has the function equivalent to the substances above.

### REFERENCE SIGNS LIST

- 1: light scattering particle measuring apparatus
- 2: light source
- 3: incident optical system
- 4: sample cell
- 5: outgoing optical system
- 6: light receiving element
- 7: amplifier circuit
- 8: noise removing filter
- 9: arithmetic and logic unit
- 10: indicator
- 11: stirring bar
- 12: stirrer
- 21: albumin molecule
- 22: chain-shaped fragment by albumin
- 23: endotoxin
- 24: coagulin gel

## Claims

1. A method of measuring an organism-originated physiologically active substance to measure a concentration of a physiologically active substance in a hyaluronic acid preparation by allowing the organism-originated physiologically active substance present in the hyaluronic acid preparation to react with an AL of a hemocyte extract of horseshoe crab **characterized in that**,
an aqueous electrolyte solution for dilution and a predetermined protein exhibiting higher affinity for the organism-originated physiologically active substance than hyaluronic acid are included in a mixture of an AL reagent containing the AL and the hyaluronic acid preparation.

2. The method of measuring an organism-originated physiologically active substance according to claim 1, wherein the predetermined protein is albumin.

3. The method of measuring an organism-originated physiologically active substance according to claim 1 or 2, wherein the aqueous electrolyte solution for dilution is a phosphate buffered saline.

4. The method of measuring an organism-originated physiologically active substance according to any one of claims 1 to 3, wherein a pH of the mixture of the AL reagent and the hyaluronic acid preparation in a state of including the aqueous electrolyte solution for dilution is 5.5 or higher.

5. The method of measuring an organism-originated physiologically active substance according to any one of claims 1 to 4, wherein the organism-originated physiologically active substance is endotoxin or β-D-glucan.
